# EUROPEAN PATENT APPLICATION

(11) **EP 4 763 093 A1**
(43) Date of publication of application: **24.06.2026**
(21) Application number: 24221411.2
(22) Date of filing: 19.12.2024
(51) Int. Cl.: A61B 8/00, A61B 8/08

(54) **PROCESSING ULTRASOUND IMAGES**

(71) Applicant: Koninklijke Philips N.V., 5656 AE Eindhoven (NL)
(72) Inventor: WEHLE, Simon, Eindhoven (NL); WAECHTER-STEHLE, Irina, Eindhoven (NL); GESSERT, Nils Thorben, 5656AG Eindhoven (NL); SCHULZ, Heinrich, Eindhoven (NL)
(74) Representative: Philips Intellectual Property & Standards

(57) **Abstract**

The present disclosure provides concepts for processing an ultrasound image stream. These concepts include obtaining ultrasound images of the heart including at least one set of consecutive ultrasound images corresponding to a heart cycle sequence. For each ultrasound image in the set of consecutive ultrasound images, a cardiac view included in the ultrasound image is determined. A transition value is generated indicating whether the cardiac view included in the ultrasound images has switched during the heart cycle sequence based on the determined cardiac views associated with the consecutive set of ultrasound images. Using this transition value, it may be quickly and automatically determined whether the set of ultrasound images are suitable for subsequent assessment.

## Description

### FIELD OF INVENTION

The present invention relates to processing ultrasound image streams of the heart, and in particular, to systems and methods for analyzing cardiac views and transitions between views during heart cycle sequences.

### BACKGROUND OF INVENTION

Ultrasound imaging is a widely used medical diagnostic technique that provides real-time visualization of internal body structures, including the heart. In cardiac ultrasound, also known as echocardiography, different views of the heart are obtained to assess its structure and function. These views, such as the parasternal long axis, apical four-chamber, and subcostal views, provide crucial information for diagnosing various cardiac conditions.

During an echocardiographic examination, sonographers often need to switch between different cardiac views to comprehensively evaluate the heart. However, this process can be challenging, especially when dealing with complex cardiac abnormalities or in subjects with difficult acoustic windows. The transition between views may result in momentary loss of image quality or orientation, potentially leading to missed diagnoses or prolonged examination times.

Furthermore, the interpretation of cardiac ultrasound images requires significant expertise and experience. Sonographers and cardiologists must be able to quickly identify the specific cardiac view being displayed and assess its clinical relevance. This task becomes particularly demanding when reviewing large volumes of ultrasound data or when performing real-time analysis during procedures. In particular, during a stress echo exam, the sonographer may have to collect various heart cycle sequences (i.e., cine loops) of various cardiac views within 1 to 2 minutes whilst the subject is under peak stress (e.g., due to exercise or the administration of a stimulating substance). Whilst continuous acquisitions can speed up this process, it still requires sequence splitting after the exam, and there is little guarantee that each cardiac view is adequately imaged.

Accordingly, there exists a need for an improved means for cardiac ultrasound image analysis to overcome one or more of these problems.

### SUMMARY OF INVENTION

The invention is defined by the claims.

According to an aspect of the present disclosure, a method for is provided for processing an ultrasound image stream.

The method comprises: obtaining ultrasound images of the heart including at least one set of consecutive ultrasound images corresponding to a heart cycle sequence; for each ultrasound image in the set of consecutive ultrasound images, determining a cardiac view included in the ultrasound image; and generating a transition value indicating whether the cardiac view included in the ultrasound images has switched during the heart cycle sequence based on the determined cardiac views associated with the consecutive set of ultrasound images.

In other words, there is provided a means for processing ultrasound image streams of the heart, particularly focusing on analyzing cardiac views and transitions between views during heart cycle sequences. By leveraging information regarding the cardiac views present in images in a single heart cycle sequence, it can be automatically identified whether a transition in the cardiac view has occurred. That is, if multiple different cardiac views are identified in a set of consecutive ultrasound images, then this implies that there has been a transition. Thus, the set of images may be unsuitable for assessment of the heart.

This method therefore facilitates automated detection of changes in cardiac views during a heart cycle, which can improve the efficiency and accuracy of ultrasound image analysis, particularly in identifying potential imaging errors or anomalies. Building upon this view identification, the invention generates a transition value. This value indicates whether the cardiac view has switched during the heart cycle sequence. The transition value is generated based on the determined cardiac views associated with the consecutive set of ultrasound images. This approach allows for the detection and quantification of view changes, which can be crucial in identifying potential issues in image acquisition or subject positioning.

By automating the process of cardiac view identification and transition detection, this invention offers several advantages. It can significantly reduce the cognitive load on sonographers and cardiologists, potentially improving the speed and accuracy of echocardiographic examinations. The system's ability to flag or discard image sets with excessive view transitions can enhance the overall quality of acquired data. Furthermore, the real-time processing capability opens up possibilities for immediate feedback during image acquisition, potentially leading to more efficient and effective ultrasound examinations.

In some embodiments, the obtained ultrasound images may include a plurality of sets of consecutive ultrasound images, with each set corresponding to a different heart cycle sequence. For each ultrasound image in each set of consecutive ultrasound images, a cardiac view included in the ultrasound image may be determined, and for each set of ultrasound images, a transition value may be generated indicating whether the cardiac view included in the ultrasound images has switched during the heart cycle sequence based on the determined cardiac views associated with the ultrasound images included in the respective set.

This approach enables analysis of multiple heart cycles, providing a more comprehensive assessment of cardiac function and potentially identifying patterns or anomalies that may not be apparent from a single heart cycle. This facilitates the automated splitting of a sequence of ultrasound images, to generate sets corresponding to different heart cycles that are then assessed to determine whether a transition has occurred during the acquisition of the images in the heart cycle sequence.

In other words, at present a sonographer must manually split an ultrasound image sequence, usually after the examination has concluded. This process is time consuming and tedious. In addition, as the splitting does not occur during the examination, it may be difficult to determine during the examination whether a sequence of images of the heart during a full heart cycle sequence for each desired cardiac view has been successfully captured (until the subsequent assessment has occurred). This embodiment overcomes this issue by automatically splitting and assessing the sequence of images.

The method may further comprise processing the obtained ultrasound images to identify the plurality of sets of consecutive ultrasound images. This may involve obtaining electrocardiogram (ECG) data of the heart acquired simultaneously to the ultrasound images, wherein identifying the plurality of sets of consecutive ultrasound images is based on the obtained ECG data.

This allows for accurate splitting of the ultrasound image stream into distinct heart cycles, improving the reliability of subsequent analysis. As long as the ECG data and ultrasound images are appropriately synchronized, this may allow for a computationally simple and highly accurate way to divide the ultrasound images into sets.

Alternatively, identifying the plurality of sets of consecutive ultrasound images may comprise processing the ultrasound images with an image analysis algorithm.

Indeed, this also allows for accurate splitting of the ultrasound image stream into distinct heart cycles, improving the reliability of subsequent analysis. Features in the images themselves may be leveraged to divide the plurality of images without the need for other data sources, which may suffer from a wider variety of errors and may require appropriate synchronization with the image acquisition.

juIn some embodiments, determining the cardiac view included in each ultrasound image may comprise processing the ultrasound image with a view identification machine learning model to generate likelihood values, each likelihood value indicating a probability that a different cardiac view is included in the ultrasound image, and determining the cardiac view based on the generated likelihood values.

This machine learning approach can enhance the accuracy and efficiency of cardiac view identification, potentially detecting subtle features that may be challenging for human observers to consistently recognize. Furthermore, the machine learning approach provides a plurality of likelihood values (i.e., probabilities) that certain cardiac views are present in each image. It may not be entirely certain whether individual ultrasound images relate to one particular cardiac view, and therefore a likelihood value provides an accurate way of reflecting the confidence that a particular cardiac view is presented.

Generating the transition value may be based on the likelihood values associated with the consecutive set of ultrasound images.

The more granular information provided by the likelihood values (as opposed to a simplified determination of a single cardiac view included in the image) provides for a more meaningful generation of a transition value that can reflect the actual chance that a transition has occurred in cardiac view in a single heart cycle sequence.

More specifically, this may involve, for each cardiac view, calculating an average of the likelihood values associated with the respective cardiac view included in the consecutive ultrasound images; for each cardiac view, calculating a standard deviation of the likelihood values associated with the respective cardiac view included in the consecutive ultrasound images; and generating the transition value based on the calculated averages and standard deviations of each of the cardiac views.

This statistical analysis of likelihood values provides a robust method for detecting transitions between cardiac views, accounting for natural variations in image quality and cardiac motion. In this way, random errors in detection and/or variances in imaging conditions may not lead to a conclusion that there has been a transition in a set of ultrasound images. Equally, if a pattern of variance is detected then it may be more likely that a change in the cardiac view has occurred during the heart cycle sequence.

Alternatively, generating the transition values may comprise processing the likelihood values associated with the consecutive set of ultrasound images with a transition analysis machine learning model.

Utilizing a machine learning model for transition analysis can potentially capture complex patterns in view transitions that may be difficult to define with explicit rules, improving the overall accuracy of the method.

The method may further comprise generating a stability value based on the likelihood values, the stability value indicating a stability of visualization of a cardiac view of the heart included in the set of consecutive ultrasound images.

This stability assessment can help identify periods of consistent imaging, which may be particularly valuable for subsequent quantitative analysis or diagnosis. Indeed, it has been noticed that variances in likelihood values can be indicative of an unstable acquisition.

Responsive to the transition value meeting a predetermined condition, the method may include discarding the set of consecutive ultrasound images and/or flagging the set of consecutive ultrasound images.

This feature allows for automatic quality control, potentially reducing errors in subsequent analysis by identifying and managing problematic image sequences. This may allow a sonographer to quickly and easily understand when a set of ultrasound images throughout a heart cycle sequence need to be recaptured.

In some embodiments, the ultrasound images may be obtained in real-time from an ultrasound imaging system, enabling immediate processing and feedback during ultrasound examinations. Nevertheless, in other embodiments of the invention the ultrasound images may be processed after the examination has concluded.

According to another aspect of the present disclosure, a computer program comprising computer program code means adapted, when said computer program is run on a computer, to implement the method of processing an ultrasound image stream is provided.

According to another aspect of the present disclosure, a system for processing an ultrasound image stream is provided.

The system comprises: an interface configured to obtain ultrasound images of the heart including at least one set of consecutive ultrasound images corresponding to a heart cycle sequence; a processor configured: for each ultrasound image in the set of consecutive ultrasound images, to determine a cardiac view included in the ultrasound image; and to generate a transition value indicating whether the cardiac view included in the ultrasound images has switched during the heart cycle sequence based on the determined cardiac views associated with the consecutive set of ultrasound images.

These and other aspects of the invention will be apparent from and elucidated with reference to the embodiment(s) described hereinafter.

### BRIEF DESCRIPTION OF THE DRAWING

For a better understanding of the invention, and to show more clearly how it may be carried into effect, reference will now be made, by way of example only, to the accompanying drawings, in which:
Fig. 1 illustrates a flowchart of a method for processing an ultrasound image stream according to an embodiment;
Fig. 2 illustrates a flowchart of a method for splitting an ultrasound image stream according to aspects of the present disclosure;
Fig. 3 illustrates a block diagram of a system for processing an ultrasound image stream according to another embodiment; and
Fig. 4 is a simplified block diagram of a computer within which one or more parts of an embodiment may be employed.

### DETAILED DESCRIPTION OF EMBODIMENTS

The invention will be described with reference to the Figures.

It should be understood that the Figures are merely schematic and are not drawn to scale. It should also be understood that the same reference numerals are used throughout the Figures to indicate the same or similar parts.

It should also be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to provide an advantage.

The present disclosure provides concepts for processing an ultrasound image stream. These concepts include obtaining ultrasound images of the heart including at least one set of consecutive ultrasound images corresponding to a heart cycle sequence. For each ultrasound image in the set of consecutive ultrasound images, a cardiac view included in the ultrasound image is determined. A transition value is generated indicating whether the cardiac view included in the ultrasound images has switched during the heart cycle sequence based on the determined cardiac views associated with the consecutive set of ultrasound images. Using this transition value, it may be quickly and automatically determined whether the set of ultrasound images are suitable for subsequent assessment.

In particular, there are provided methods and systems for processing ultrasound image streams, particularly for analyzing cardiac views and transitions during heart cycle sequences. Ultrasound imaging is a widely used technique for visualizing the heart and assessing cardiac function. However, the interpretation of ultrasound images can be complex and time-consuming, especially when analyzing multiple images over a heart cycle.

A cardiac ultrasound examination may be performed when it is suspected that a subject may suffer from heart disease, such as coronary artery disease. In some cases, a stress exam may be performed, particularly when results from the standard cardiac ultrasound examination are relatively normal. During the stress exam, ultrasound images are acquired in the subject's rest phase. Then stress is induced, either pharmacologically or by exercise. Ideally, images are acquired at the peak of the stress. In both cases, sequences of the same standard cardiac view planes are acquired. After the examination corresponding images are compared.

Unfortunately, ultrasound image quality of a stress exam is often lower than a standard exam, as there is more motion and less time to perform image acquisition. Image acquisition must be performed within 1 to 2 minutes while the subject is under peak stress.

During these 1 to 2 minute periods, the sonographer must position and maneuver the ultrasound probe, identify which cardiac view is being imaged by the probe, trigger the acquisition of a cine-loop (i.e., trigger acquisition of a set of consecutive ultrasound images corresponding to a single heart cycle sequence), and then decide whether the quality of the set of images is sufficient. This process must be completed for all desired cardiac views, such as apical 2/3/4 chambers, PLAX, and SAX. Continuous acquisitions can speed up this process (not requiring the sonographer to trigger the acquisition of the cine-loop). However, this requires sequence splitting afterward, which is a time consuming and tedious task for a sonographer.

Embodiments of the proposed invention therefore provide a way to efficiently process ultrasound image streams to determine cardiac views and detect transitions between different views. This approach involves obtaining ultrasound images of the heart, including at least one set of consecutive ultrasound images corresponding to a heart cycle sequence. For each ultrasound image in the set, a cardiac view included in the image is identified/determined. This determination allows for the generation of a transition value that indicates whether the cardiac view included in the ultrasound images has switched during the heart cycle sequence. If the cardiac view has switched, it is likely of little use for subsequent analysis. This set may therefore be simply discarded, and/or a user may be notified that the set is inadequate.

To be clear, the transition value is based on the determined cardiac views associated with the consecutive set of ultrasound images. By automatically detecting view transitions, the method can identify potential issues in image acquisition or highlight clinically relevant changes in cardiac structure or function.

This automated approach to analyzing quality of cardiac ultrasound image streams can significantly enhance the efficiency and accuracy of cardiac assessments. The method may be particularly useful in clinical settings where there is little time to acquire the sets of ultrasound images (e.g., during stress exams, or when staffing shortages are a concern) and/or where rapid analysis of large volumes of ultrasound data is required.

More specifically, embodiments may address the challenges of manual sequence splitting and view detection during a cardiac ultrasound examination through a combination of some or all of the following features:
- Cardiac view detection. For example, there may be provided a machine learning algorithm automatically identifies and labels different cardiac views (apical 2/3/4 chambers, PLAX, SAX, etc.) in ultrasound images. This may be performed in real-time during continuous acquisition of the ultrasound images;
- Automatic sequence splitting. For example, ECG data may be used to split the continuous acquisition of ultrasound images into sets of ultrasound images, each set corresponding to distinct heart cycle sequences (heart cycle clips). The cardiac view of the set may be labelled automatically based on the result of the cardiac view detection. This eliminates the need for manual intervention.
- Transition detection. Heart cycles during which the sonographer switches between different cardiac views (i.e., sets of ultrasound images during which the sonographer switched cardiac views, and therefore different ultrasound images in the set include different cardiac vies) are identified.
- Quality grading and sorting. For example, each set of ultrasound images may be graded based on a transition value (indicating the probability of the cardiac view being the same in all ultrasound images in the set) and/or a stability value describing a quality of the set of ultrasound images. Accordingly, the sets of ultrasound images (i.e., the clips) may be sorted for easier review and analysis.
- Feedback. Real-time feedback may be provided, marking sets of ultrasound images that are unusable due to cardiac view transitions. This may help to affect immediate correction and adjustment.

Accordingly, embodiments of the invention may provide a means for accurately, reliably and automatically processing an ultrasound image stream to identify sets of ultrasound images (each corresponding to a single heart cycle sequence) throughout which a same cardiac view is present. The identification of when this is not the case (i.e., when the cardiac view captured in the ultrasound images has changed within a heart cycle sequence) allows for the deletion of unusable sets of ultrasound images, and/or notification to the sonographer that the acquisition is unusable. This may ensure that the ultrasound examination is performed speedily and completely.

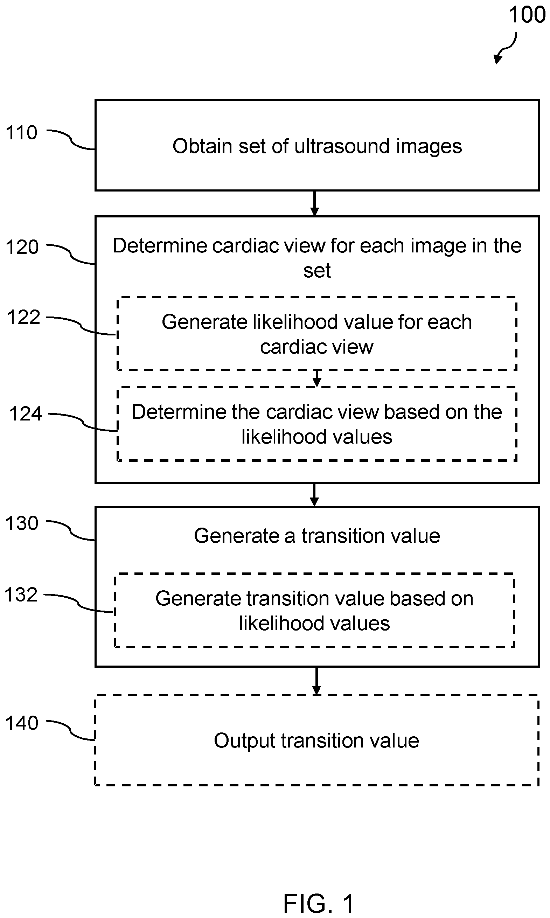

Fig. 1 illustrates a flowchart of a method for processing an ultrasound image stream according to an embodiment.

In step 110, a set of ultrasound images are obtained. The set of ultrasound images correspond to a heart cycle sequence. That is, each of the ultrasound images in the set may include the heart of the subject during the same heartbeat of the subject. A set of ultrasound images may be referred to as a cine-loop, or a clip.

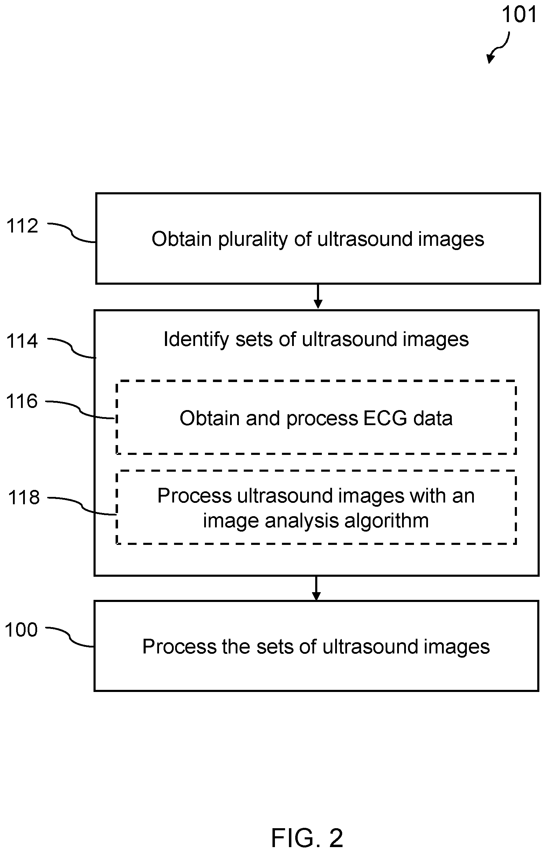

In reference to Fig. 2, methods are discussed wherein a plurality of ultrasound images are first obtained comprising a plurality of different sets of ultrasound images, each corresponding to different heart cycle sequences. However, for simplicity, we first consider the processing of only a single set of ultrasound images corresponding to a single heart cycle sequence (thereby avoiding the need for a step of splitting the ultrasound images into sets) in reference to Fig. 1. This may occur, for example, when a plurality of ultrasound images have been pre-processed to provide sets of ultrasound images.

Following the acquisition of the set of ultrasound images, in step 120, the cardiac view included in each ultrasound image in the set is determined. That is, for each ultrasound image in the set, a determination is made as to which cardiac view is presented by the respective image.

The determination may be made by a view identification machine learning model. This model is an AI model trained to identify/predict cardiac views in ultrasound images. To this end, it may have been trained on previously acquired ultrasound images of the heart labelled with the cardiac view contained in the ultrasound images, using supervised or unsupervised training techniques. For example, the view identification machine learning model may be a convolutional neural network (CNN), which is particularly suitable for computer vision applications.

In sub-step 122, likelihood values are generated for each cardiac view for each image. In particular, the view identification machine learning model may be configured to generate a plurality of likelihood values. Each likelihood value may describe the probability that a certain cardiac view is included in the ultrasound image. That is, the view identification machine learning model produces a plurality of likelihood values, each corresponding to a different cardiac view, and describing a probability that the respective cardiac view is present in the ultrasound image. As a result, if the view identification machine learning model predicts with high confidence/certainty that a particular cardiac view is included in an ultrasound image, it will generate a high likelihood value for that cardiac view, and low likelihood values for other cardiac views. In contrast, if the view identification machine learning model cannot predict the presence of any cardiac view with a high level of confidence/certainty then low likelihood values will be generated for many cardiac views.

In other words, the view identification machine learning model may be configured to generate a probability distribution for the cardiac views per ultrasound image in the set of ultrasound images.

In sub-step 124, the cardiac view for each image is determined based on the generated likelihood values. This may be a straightforward manner of selecting the cardiac view with the highest associated likelihood value for each ultrasound image. In other cases, the likelihood values for immediately preceding or subsequent ultrasound images may be considered. In yet further cases, the probability distribution of the cardiac views for each image may be considered. For example, the determination may be made based on a statistical analysis, or by providing the likelihood values to a machine learning model.

In step 130, a transition value is generated for the set of ultrasound images. The transition value indicates whether the cardiac view included in the ultrasound images in the set has switched during the heart cycle sequence of the subject based on the determined cardiac views associated with the each of the ultrasound images.

In some cases, the transition value may be a binary value - either indicating that there has been a change in cardiac view in the set of ultrasound images, or not. In other cases, however, the transition value may indicate a degree in confidence as to whether there has been a change in cardiac view in the set. This would provide a level of granularity, as it may not be completely clear as to whether a change has occurred. Nevertheless, this information may be relevant for deciding whether the set is clinically useful.

In the case that likelihood values have been generated for each cardiac view for each ultrasound image (i.e., a probability distribution for the cardiac views per ultrasound image in the set of ultrasound images has been generated), then the transition value may be based on the likelihood values associated with the consecutive set of ultrasound images.

Put another way, if likelihood values for the cardiac views in each ultrasound image in the set have been generated, the transition value may be generated based on said likelihood values. To this end, the likelihood values may be subject to a statistical or analytical method to determine the probability of a transition of the cardiac view in the set of ultrasound images.

More specifically, for each cardiac view, an average of the likelihood values associated with the respective cardiac view included in the consecutive ultrasound images may be calculated. For example, the likelihood values associated with a PLAX view for each of the ultrasound images in the set may be averaged. Then, the likelihood values associated with a SAX view for each of the ultrasound images in the set may be averaged, and so on for each cardiac view of interest. Clues are provided as to whether there has been a transition by these averages. Accordingly, the transition value may be generated based on the calculated averages.

For example, if two different cardiac views have averages above a threshold, then this may indicate that these two cardiac views are present in the set, and therefore it is likely a transition has occurred. In contrast, if only one cardiac view has a likelihood value average above a threshold, this may indicate that a transition has not occurred.

In addition, for each cardiac view, a standard deviation of the likelihood values associated with the respective cardiac view included in the consecutive ultrasound images may be calculated. That is, a variance in the likelihood values of cardiac views between images may be assessed. This may provide additional clues as to whether there has been a transition. Accordingly, the transition value may be generated based on the calculated standard deviation of each of the cardiac views (and perhaps most importantly on the standard deviation of the cardiac view with the highest associated calculated average).

For example, if the standard deviation of the likelihood values associated with the cardiac view across the ultrasound images is low, then this may indicate consistency between ultrasound images and therefore a small chance of a transition. Equally, a high standard deviation may indicate inconsistency between images and therefore a high chance of a transition.

Of course, further statistical analysis (e.g., a slope or regression analysis) may also be performed and may provide an indication as to whether there has been a transition. Such analysis may therefore be used to alter the transition value. Additional or alternative ways of processing the likelihood values are also envisioned. Indeed, in one embodiment, a transition analysis machine learning model may be used to analyse the likelihood values associated with the consecutive set of ultrasound images, in order to generate the transition value. Each of these techniques may be used to enhance transition detection.

In yet further embodiment of the invention, the method may further comprise generating a stability value. This stability value indicates the stability of visualization of a cardiac view of the heart included in the set of consecutive ultrasound images. The stability value may also be based on the likelihood values.

For example, a relatively low highest average likelihood value of the set of ultrasound images may indicate a degree of uncertainty as to the cardiac view included in the ultrasound image set. This may be due to a poor stability of visualization, as the cardiac view detection is uncertain. Equally, a high standard deviation of the likelihood values may also indicate a degree of uncertainty as to the cardiac view included in the ultrasound image set, as this may imply a difference in visualization of each of the ultrasound images in the set (which leads to a difference in certainty as to the cardiac view included in each ultrasound image).

To summarize briefly, the use of likelihood values in determining cardiac views and/or generating transition values provides a probabilistic approach to analyzing the ultrasound images. This approach may enhance the accuracy of cardiac view identification and the detection of transitions between views. The optional stability value offers additional information about the consistency of cardiac view visualization throughout the heart cycle sequence.

Finally, in step 140, the transition value is output. The transition value may simply be stored for future use or may be output to a user for assessment to determine further action to be taken regarding the set of ultrasound images. Usually it is desired (for subsequent analysis) that each of the ultrasound images in the set include the same cardiac view (e.g., each ultrasound image includes an apical 2/3/4 chambers view, a PLAX view, a SAX view of the heart, etc.). Accordingly, if the transition value is high, then the user may choose to discard the set of ultrasound images, recapture the set of ultrasound images, and/or make appropriate adjustments.

In further embodiments, the transition value may be used to automatically process the set of ultrasound images. For example, responsive to the transition value meeting a predetermined condition (e.g., exceeding a certain value), the set of consecutive ultrasound images may be discarded. Alternatively, or additionally, the set of consecutive ultrasound images may be flagged to indicate to a user that a transition is likely to have occurred.

Fig. 2 illustrates a flowchart of a method for splitting an ultrasound image stream according to aspects of the present disclosure. As discussed above, the method for processing the ultrasound images may be applied to a plurality of undivided ultrasound images, not just a single set of ultrasound images corresponding to one heart cycle sequence. However, to achieve this, splitting/dividing/grouping the plurality of ultrasound images must be achieved before the method of Fig. 1 can be applied.

In step 112, a plurality of ultrasound images are obtained. The plurality of ultrasound images may be acquired in real-time from an ultrasound device, for example. This may enable processing of the ultrasound images in real-time, and therefore may provide fast feedback to a user. Nonetheless, the ultrasound images may be obtained for storage (e.g., from electronic medical records) in order to perform post-acquisition analysis.

In step 114, sets of consecutive ultrasound images are identified. Each set of consecutive ultrasound images corresponds to different heart cycle sequences. That is, each set of ultrasound images comprises images of the heart during a single heart cycle sequence (e.g., during a single heartbeat). Different sets of ultrasound images correspond to different heart cycle sequences.

Essentially, step 114 involves the separation of the plurality of ultrasound images into the sets of consecutive ultrasound images. To achieve this, the timing of the heart cycle sequences must be known or deduced. From this information, the plurality of ultrasound images can be split into the sets of ultrasound images.

To this end, the method may comprise step 116. In step 116, electrocardiogram (ECG) data of the heart that was acquired simultaneously to the ultrasound images is obtained. The ECG data may be obtained directly from an ECG device fitted to the subject during the ultrasound examination, or may be obtained from storage (e.g., electronic medical records). ECG information contains information suitable for identifying the phase of the heart cycle sequence that the heart is in at any given moment. Accordingly, the plurality of sets of consecutive ultrasound images may be identified based on the obtained ECG data.

Alternatively, the method may comprise step 118. In step 118, each of the ultrasound images are processed with an image analysis algorithm. This algorithm may identify the phase of the heart cycle sequence is in during acquisition of the ultrasound image based on information within the ultrasound image. Indeed, the shape of the heart indicates the phase of the heart cycle sequence and can be used to differentiate between heart cycles. Accordingly, the plurality of sets of consecutive ultrasound images may be identified based on the result of processing the ultrasound images with the image analysis algorithm.

Of course, steps 116 and 118 may be used in combination to identify the sets of ultrasound images. Either the ECG data or the output from the image analysis algorithm may be used to differentiate between heart cycle sequences, and the other may be used to verify the conclusion, for example. In addition, other methods and sources of information used to identify the sets of ultrasound images may be contemplated.

Then, the method proceeds to the method 100 for processing the sets of ultrasound images of Fig. 1. Accordingly, each identified set of ultrasound images may be analyzed, so as to identify cardiac views in images within the set and generate a transition value for each set of ultrasound images. Thus, a full analysis of the ultrasound image stream may be realized.

In some cases, the sets of ultrasound images may then be sorted according to their associated transition values and determined cardiac views. For example, the set of ultrasound images for each cardiac view with the lowest transition values may be identified. These may be considered the most appropriate sets for further analysis. Accordingly, these may automatically be provided to a user for subsequent analysis. Alternatively, sets of ultrasound images having high transition values may be deleted, and only the sets of ultrasound images having low transition values may be stored, output and/or presented to the user.

Accordingly, the described methods provide a means for reliably, efficiently and automatically processing ultrasound images in order to identify sets of ultrasound images that may be unsuitable for further analysis (e.g., due to a change in cardiac view that has occurred during the heart cycle sequences associated with the set). This may be useful during continuous acquisition of the ultrasound images, particularly when the operator is under time constraints, as it facilitates real-time feedback.

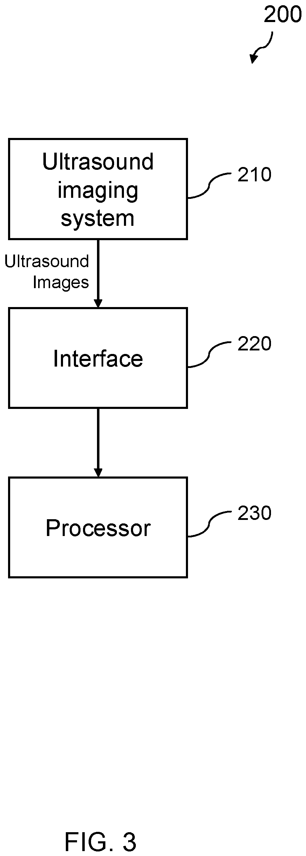

Fig. 3 illustrates a block diagram of a system for processing an ultrasound image stream according to another embodiment. The ultrasound processing system 200 comprises an ultrasound imaging system 210, an interface 220, and a processor 230.

The ultrasound imaging system 210 generates ultrasound images of the heart. These ultrasound images may include at least one set of consecutive ultrasound images corresponding to a heart cycle sequence. The ultrasound imaging system 210 may be any suitable ultrasound device capable of capturing cardiac images. In other embodiments, the ultrasound imaging system 210 may not be provided. Instead, the ultrasound image stream may be obtained from storage (e.g., electronic medical records).

The interface 220 is configured to obtain the ultrasound images from the ultrasound imaging system 210. The interface 220 receives and processes the incoming image data, preparing the ultrasound images for further analysis. The interface 220 may include hardware and software components designed to facilitate the transfer and initial processing of ultrasound image data.

The processor 230 is connected to the interface 220 and performs processing operations on the received ultrasound images. The processor 230 is configured to implement the methods described in previous figures, including determining a cardiac view included in each ultrasound image in the set of consecutive ultrasound images.

Additionally, the processor 230 is configured to generate a transition value indicating whether the cardiac view included in the ultrasound images has switched during the heart cycle sequence. This transition value is based on the determined cardiac views associated with the consecutive set of ultrasound images.

In some cases, the transition value may then be output to a user interface. The user interface may be configured to provide a user with an indication of the transition value. In other cases, the transition value may be used to automatically process the ultrasound image set (e.g., delete or store the set as appropriate). Alternatively, the transition value may simply be stored for future reference.

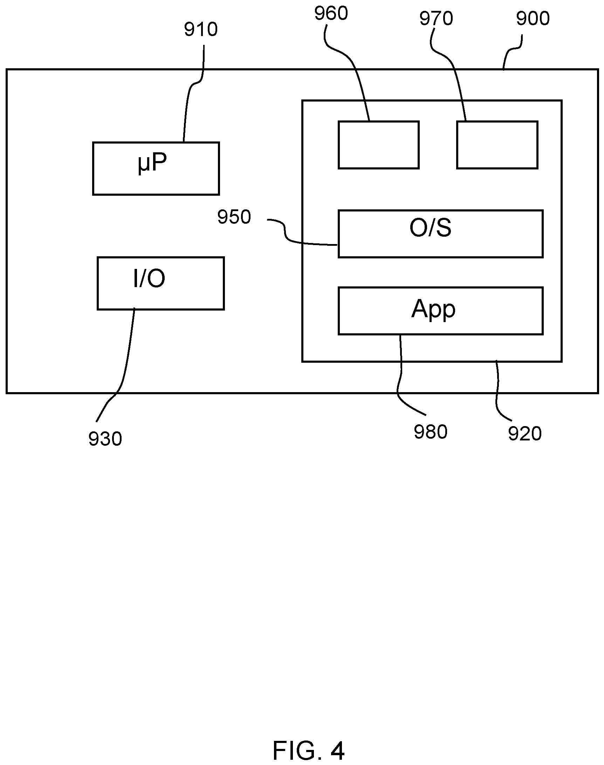

Fig. 4 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc.

Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to provide an advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A method for processing an ultrasound image stream, comprising:
obtaining (110) ultrasound images of the heart including at least one set of consecutive ultrasound images corresponding to a heart cycle sequence;
for each ultrasound image in the set of consecutive ultrasound images, determining (120) a cardiac view included in the ultrasound image; and
generating (130) a transition value indicating whether the cardiac view included in the ultrasound images has switched during the heart cycle sequence based on the determined cardiac views associated with the consecutive set of ultrasound images.

2. The method of claim 1, wherein the obtained ultrasound images include a plurality of sets of consecutive ultrasound images, each set corresponding to a different heart cycle sequence.

3. The method of claim 2, further comprising:
for each ultrasound image in each set of consecutive ultrasound images, determining (120) a cardiac view included in the ultrasound image;
for each set of ultrasound images, generating a transition value (130) indicating whether the cardiac view included in the ultrasound images has switched during the heart cycle sequence based on the determined cardiac views associated with the ultrasound images included in the respective set.

4. The method of claim 2 or 3, further comprising processing the obtained ultrasound images to identify (114) the plurality of sets of consecutive ultrasound images.

5. The method of claim 4, further comprising obtaining (116) electrocardiogram, ECG, data of the heart acquired simultaneously to the ultrasound images, and wherein identifying (114) the plurality of sets of consecutive ultrasound images is based on the obtained ECG data.

6. The method of claim 4, wherein identifying (114) the plurality of sets of consecutive ultrasound images comprises processing (118), with an image analysis algorithm, the ultrasound images.

7. The method of any of claims 1-6, wherein determining (120) the cardiac view included in each ultrasound image comprises processing, with a view identification machine learning model, the ultrasound image to generate likelihood values (122), each likelihood value indicating a probability that a different cardiac view is included in the ultrasound image, and wherein determining (124) the cardiac view is based on the generated likelihood values.

8. The method of claim 7, wherein generating the transition value (132) is based on the likelihood values associated with the consecutive set of ultrasound images.

9. The method of claim 8, wherein generating (130) the transition values comprises:
for each cardiac view, calculating an average of the likelihood values associated with the respective cardiac view included in the consecutive ultrasound images;
for each cardiac view, calculating a standard deviation of the likelihood values associated with the respective cardiac view included in the consecutive ultrasound images; and
generating the transition value (132) based on the calculated averages and standard deviations of each of the cardiac views.

10. The method of any of claims 7-9, wherein generating (132) the transition values comprises processing, with a transition analysis machine learning model, the likelihood values associated with the consecutive set of ultrasound images.

11. The method of any of claims 7-10, further comprising generating a stability value based on the likelihood values, the stability value indicating a stability of visualization of a cardiac view of the heart included in the set of consecutive ultrasound images.

12. The method of any of claims 1-11, further comprising, responsive to the transition value meeting a predetermined condition, discarding the set of consecutive ultrasound images and/or flagging the set of consecutive ultrasound images.

13. The method of any of claims 1-12, wherein the ultrasound images are obtained in real-time from an ultrasound imaging system.

14. A computer program comprising computer program code means adapted, when said computer program is run on a computer, to implement the method of any of claims 1-14.

15. A system (200) for processing an ultrasound image stream, the system comprising:
an interface (220) configured to obtain ultrasound images of the heart including at least one set of consecutive ultrasound images corresponding to a heart cycle sequence;
a processor (230) configured:
for each ultrasound image in the set of consecutive ultrasound images, to determine a cardiac view included in the ultrasound image; and
to generate a transition value indicating whether the cardiac view included in the ultrasound images has switched during the heart cycle sequence based on the determined cardiac views associated with the consecutive set of ultrasound images.
